# FASCICULE DE BREVET EUROPEEN

(11) **EP 3 197 506 B1**
(45) Date de publication et mention de la délivrance du brevet: **19.01.2022**
(21) Numéro de dépôt: 15778379.6
(22) Date de dépôt: 25.09.2015
(51) Int. Cl.: A61L 2/14, A61B 1/12

(54) **PROCÉDÉ DE SÉCHAGE DE CANAUX D'UN ENDOSCOPE**
VERFAHREN ZUM TROCKNEN DIE KANÄLE EINES ENDOSKOPS
METHOD FOR DRYING THE CHANNELS OF AN ENDOSCOPE

(30) Priorité: 25.09.2014 FR 1459071
(43) Date de publication de la demande: 02.08.2017
(73) Titulaire: Plasmabiotics, 95106 Argenteuil Cedex (FR)
(72) Inventeur: VINTELER, Daniel, F-92130 Issy Les Moulineaux (FR)
(74) Mandataire: Icosa
(86) Numéro de dépôt international: PCT/FR2015/052557
(87) Numéro de publication internationale: WO 2016/046503

(56) Documents cités:
- EP-B1- 2 349 597
- WO-A1-02/070025
- WO-A2-2005/000366
- FR-A1- 2 705 896
- FR-A1- 2 790 962
- FR-A1- 2 843 028
- US-A- 5 882 589
- US-A1- 2006 283 483
- US-A1- 2009 229 632

## Description

La présente invention se rapporte à un procédé rapide de séchage de canaux d'un endoscope.

L'endoscopie est une technique d'imagerie médicale largement utilisée aujourd'hui, notamment en raison de sa facilité de réalisation, de sa précision et de son faible côté invasif. Les endoscopes sont ainsi utilisés soit pour établir un diagnostic (endoscopie diagnostique), soit pour traiter une maladie ou un traumatisme (endoscopie opératoire). Leur manipulation et leur nettoyage sont cependant très spécifiques : le nettoyage et la désinfection sont nécessaires.

Classiquement, l'endoscope est nettoyé immédiatement après l'examen, avec un détergent non abrasif adapté, puis rincé. Le temps total de cette étape de nettoyage ne doit pas être inférieur à 15 minutes.

Ensuite, l'endoscope est désinfecté : il est plongé dans une solution désinfectante, puis à nouveau rincé. Enfin, il est séché partiellement à l'aide d'un pistolet à air comprimé médical. Cette dernière étape dure environ 5 minutes. Le résultat obtenu est non satisfaisant : l'étape est fastidieuse, et au final, une humidité résiduelle est toujours présente au moins dans une partie des canaux, ce qui ne garantit pas une innocuité optimale.

Pour un séchage plus efficace d'un endoscope, il existe des enceintes de stockage d'endoscopes thermosensibles (ESET). Selon les fabricants et le type d'endoscope, le séchage est réalisé entre 15 et 90 minutes.

Dans les deux cas, le procédé est long, et doit être renouvelé après chaque utilisation. Un endoscope nettoyé et non séché doit être utilisé sous une période maximale allant de 6 à 12h en France et de 3 heures maximum en Angleterre ; si ce délai est dépassé, l'endoscope doit être re-nettoyé, afin de garantir son innocuité et éviter sa recolonisation par différents pathogènes. Un délai aussi court est très contraignant, car il implique du temps, de nombreuses manipulations et des moyens humains. L'utilisation d'une ESET permet d'allonger cette période de stockage à 72 heures, grâce au séchage des canaux internes.

À titre d'exemple, le document US 2009/0229632 A1 décrit un procédé de nettoyage d'un endoscope au moyen d'une injection pulsée d'un mélange biphasique de gaz sous pression et d'une solution de nettoyage. Un tel nettoyage est suivi d'un séchage obtenu par injection, dans les canaux de l'endoscope, d'un gaz sous pression.

Il existe donc un besoin de disposer d'un procédé de séchage des endoscopes qui soit efficace, rapide, et qui n'endommage pas les endoscopes. En outre, il existe un besoin pour un procédé de séchage qui assure la sécurité microbiologique de ce type de matériel médical.

Par ailleurs, il existe un besoin pour un procédé de stockage d'endoscopes, qui soit économique, le plus automatisé possible, et qui implique un minimum de manipulations et de moyens humains. De plus le procédé doit apporter un gain de place et une réduction des consommations de gaz de séchage et d'électricité, qui sont très importantes pour les ESET.

La présente invention permet de répondre à tous ces problèmes. Notamment, la présente invention permet de sécher de manière efficace et rapide les endoscopes. Par rapport à l'état de l'art actuel, la présente invention permet notamment de gagner au moins un ordre de grandeur (facteur 10) pour le séchage des endoscopes. Elle est en outre utilisée dans des conditions compatibles avec la sensibilité des appareils. Enfin, elle permet de stocker efficacement les endoscopes nettoyés et séchés pendant une durée bien supérieure à 12h, de préférence supérieure à 72h, et ce, de façon facile et rapide.

La présente invention se rapporte donc à un procédé de séchage de canaux d'un endoscope tel que défini par la revendication 1.

Le gaz utilisé dans l'étape c) peut être neutre ou bien un plasma généré par une décharge électrique dans un flux de diazote ou bien de l'air, de préférence de l'air médical.

De préférence, la durée globale des étapes a) à c) est comprise entre 1 et 5 minutes. Cela permet donc un séchage et, lorsque le gaz est un plasma, une désinfection complémentaire ultra-rapides. La désinfection complémentaire n'est pas obligatoire mais elle peut palier un éventuel risque de contamination avec l'eau de rinçage, après une désinfection classique.

Par « nettoyage », on entend que l'endoscope a subi une opération de lavage. Cette opération peut notamment se faire manuellement ou en machine à laver. L'endoscope est alors débarrassé de souillures et prêt à subir un cycle de désinfection. De préférence dans le procédé selon l'invention, l'endoscope est préalablement nettoyé.

Par « désinfection » ou « désinfecter » un élément, on entend l'opération d'élimination volontaire et momentanée de certains germes, de manière à stopper ou prévenir une infection ou le risque d'infection ou surinfection par des micro-organismes (bactéries, protozoaires ou virus) pathogènes et/ou indésirables. La désinfection est distincte de la stérilisation, qui consiste en l'élimination définitive de certains germes.

La désinfection implique de tuer ou inactiver les micro-organismes pathogènes des éléments contaminés, en altérant leur structure ou en inhibant leur métabolisme ou certaines de leurs fonctions vitales.

La désinfection est donc un mode particulier de décontamination, i.e. ciblé sur les micro-organismes (bactéries, protozoaires et virus) pathogènes. De préférence dans le procédé selon l'invention, l'endoscope est désinfecté chimiquement préalablement aux étapes a) à c).

Par désinfection chimique, on entend une désinfection à l'aide de produits chimiques tels que les détergents enzymatiques (amylases, lipases, protéases...), les composés aminés, le glucoprotamine, l'acide peracétique et le peroxyde d'hydrogène.

L'endoscope selon l'invention est tout type d'endoscope. Il se compose d'un tube comprenant des canaux (qui sera introduit dans le corps du patient), auquel sont reliés une poignée de commande et un guide de lumière qui permet la fixation d'une caméra et de lumière.

L'endoscope peut notamment être choisi parmi :
- les bronchoscopes,
- les endoscopes digestifs, tels que les coloscopes, les gastroscopes, les duodénoscopes, et les échoendoscopes ;
- les bronchoscopes pédiatriques (qui présentent des canaux de plus petit diamètre que les bronchoscopes classiques), les uretèroscopes et les cystoscopes.

De préférence, la durée de l'étape c) est comprise entre 120 et 150 secondes pour les endoscopes digestifs, tels que les coloscopes, les gastroscopes, les duodénoscopes, et les échoendoscopes. De préférence, la durée de l'étape c) est comprise entre 1 et 2 minutes pour les bronchoscopes. Enfin, de préférence, la durée de l'étape c) est comprise entre 1 minute et 90 secondes pour les bronchoscopes pédiatriques, les cystoscopes et les uretèroscopes.

La première étape du procédé selon l'invention, i.e. l'étape a), comprend le branchement de l'endoscope à une unité de séchage afin d'injecter le gaz.

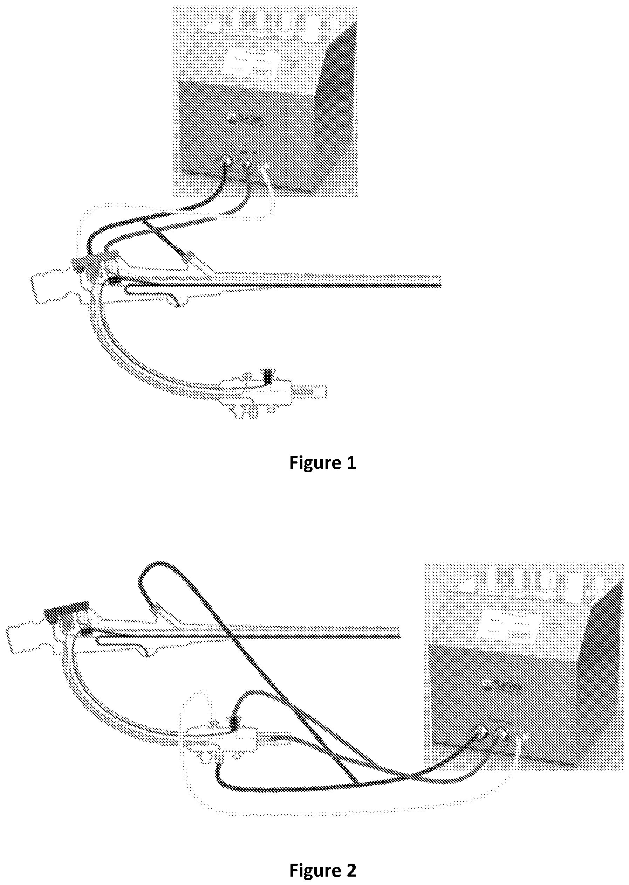

Ce branchement peut se faire soit par la cage à pistons de l'endoscope, soit par son extrémité proximale. En outre, ce branchement peut se faire par une connectique spécifique, par exemple par celle commercialisée par Lancer Getinge. Un exemple de ce branchement par la cage à piston est présenté en Figure 1, et un exemple de ce branchement par l'extrémité proximale est présenté en Figure 2. De préférence, la connectique spécifique est étanche.

Puis, après l'étape a), arrivent les étapes b) et c) : tandis que l'étape b) vise l'injection d'un gaz neutre à faible débit à une température comprise entre 10°C et 30°C, l'étape c) vise l'injection d'un gaz à fort débit à une température comprise entre 30°C et 60°C. Plus précisément, les conditions utilisées dans l'étape b) permettent un écoulement laminaire du liquide résiduel, notamment de l'eau résiduelle et du gaz de séchage, présent dans les canaux. Cet écoulement laminaire est caractérisé par un nombre de Reynolds inférieur à 2300. Cela évite la fragmentation de l'eau résiduelle et la création de gouttelettes de liquide sur les parois des canaux.

Au contraire, les conditions utilisées dans l'étape c) permettent un écoulement turbulent du gaz de séchage. Cet écoulement turbulent est caractérisé par un nombre de Reynolds très supérieur à 2300. Cela permet d'expulser et/ou évaporer la fraction de liquide restant, et assure ainsi un séchage rapide et efficace.

Précisément, lors de l'étape b), un gaz neutre est injecté dans les canaux d'endoscope pendant une durée comprise entre 10 et 60 secondes, le débit du gaz étant faible, le gaz étant injecté à une température comprise entre 10°C et 30°C, notamment pour éliminer l'eau résiduelle. Par débit faible, on entend un débit compris entre 1 et 20 1/min.

De préférence, le gaz neutre est du diazote, ou de l'air, de préférence de l'air médical. Cette étape b) est courte, i.e. quelques dizaines de secondes ; elle permet l'évacuation efficace du liquide contenu dans les canaux de l'endoscope.

Cette étape b) permet notamment d'éliminer l'eau résiduelle, notamment l'eau résultant de l'étape préalable de désinfection chimique de l'endoscope.

Enfin, le procédé selon l'invention comprend une étape c) de séchage pendant une durée comprise entre 30 et 150 secondes, de préférence comprise entre 60 et 140 secondes, par injection de gaz à fort débit à une température comprise entre 30°C et 60°C. Par fort débit, on entend un débit compris entre 20 et 100 1/min. De préférence, le gaz utilisé dans cette étape c) est un gaz neutre, préférentiellement du diazote ou de l'air. Alternativement, le gaz est un plasma généré par une décharge électrique dans un flux de diazote ou d'air.

Le séchage se fait donc par injection de gaz, et la désinfection se fait simultanément au séchage lorsque le gaz est un plasma.

De préférence, le séchage c) se fait par injection du gaz dans les canaux de l'endoscope soit par la cage à pistons, soit par son extrémité proximale.

De préférence, le plasma est obtenu par l'activation par un champ électrique, à pression atmosphérique, du flux de diazote. De préférence, le plasma est utilisé à une température comprise entre 30 et 50°C.

Les plasmas peuvent être considérés comme étant le quatrième état de la matière, en suivant par ordre croissant d'énergie les états solides, liquides et gazeux. Ce quatrième état est à proprement parler un milieu de faible densité, globalement neutre, composé d'atomes, de molécules, d'ions et d'électrons libres.

Les plasmas fabriqués par l'homme sont le plus souvent issus d'un gaz ou d'un mélange de gaz (Ar, He, air, O2, N2,...) soumis à un champ électrique (entre deux électrodes). La zone où le gaz est soumis au champ électrique est appelée zone de « décharge-électrique », le flux plasma émanant de cette décharge se trouvant dans la zone « de post-décharge ».

Classiquement, le plasma est généré par une décharge électrique (au moyen d'un champ électrique établi entre deux électrodes) dans un flux de gaz ou d'un mélange gazeux initialement inerte. On distingue deux zones de plasma : la zone de décharge et la zone de post-décharge. Dans la zone de décharge, on peut trouver des électrons, des ions, des atomes et des molécules dans différents états énergétiques. Dans la zone de post-décharge, les espèces actives retrouvées sont plutôt des atomes et des molécules neutres, qui se trouvent dans des états excités ou métastables.

De préférence, le plasma utilisé selon l'invention est un plasma froid obtenu en zone de post-décharge. De préférence, il est précisément obtenu en soumettant le flux de diazote à un champ électrique impulsionnel établi entre deux ou quatre électrodes en forme de pointes. Le champ électrique est créé par un générateur d'impulsions de haute tension (kV).

De préférence, le flux de diazote est créé en amont de son introduction dans le générateur par génération d'un flux de diazote d'un débit d'environ 1 à 100 L/min, de préférence à une pression de 1 - 2 bars. La régulation du débit des flux gazeux est effectuée à l'aide d'appareils disponibles dans le commerce, tel que le régulateur de débit Bronkhorst Mass-view.

De préférence, le plasma utilisé selon l'invention est généré comme suit :

Le diazote introduit dans le générateur traverse une chambre de décharge (réacteur) constituée d'un matériau isolant thermique résistant et stable à de très fortes températures (i.e. supérieures à 900°C, de préférence aux alentours de 1000°C). De préférence, le matériau isolant thermique résistant et stable à de très fortes températures est un mélange de céramique/verre, par exemple le matériau Macor commercialisé par Corning Inc. Un canal avec un diamètre variable, i.e. de l'ordre de quelques mm, percé à l'intérieur d'un cube en matériau isolant thermique résistant et stable à de très fortes températures, de préférence en Macor, sert pour le passage de gaz. Un ou deux canaux avec un diamètre de 1 mm sont percés perpendiculairement par rapport au canal du flux gazeux. Les électrodes en tungstène pur et en forme de pointes sont insérées dans ces canaux et scellées. La distance entre les pointes des électrodes est de quelques mm.

Une fois que le flux de diazote est établi dans le réacteur, on peut démarrer le générateur d'impulsions nanosecondes de haute tension. La haute tension (1 - 10 kV) créée par le générateur est utilisée pour établir un champ électrique entre les électrodes dans le réacteur, avec une fréquence comprise entre 10 et 100 kHz, de préférence comprise entre 30 et 80 kHz. La tension entre les électrodes augmente et une fois que la tension d'amorçage entre les électrodes est atteinte, la décharge se produit dans le réacteur. Lors de l'amorçage, la tension entre les électrodes chute très rapidement et le courant de décharge obtient la forme d'un pic d'une largeur à mi-hauteur de l'ordre de 10 ns. Le plasma créé lors de cette décharge est d'une température autour de 300-340 K (i.e. 26.85-66.85°C) et il se propage sur quelques mètres dans les tubes d'endoscopes.

De préférence, l'appareil utilisé pour générer le plasma est le générateur InPulse ONE, commercialisé par PlasmaBiotics SAS.

De préférence, le plasma selon l'invention est obtenu et utilisé à la pression atmosphérique.

Plus préférentiellement, le plasma est obtenu, dans l'unité plasma de séchage, par les étapes suivantes :
- passage d'un flux de diazote, ayant un débit d'environ 1 à 100L/min, dans l'unité ; puis
- soumission du flux obtenu à une décharge électrique.

Le procédé selon l'invention comprend la mise en contact des canaux de l'endoscope avec le plasma.

De préférence, la mise en contact se fait lorsque le plasma a une température comprise entre 30 et 50°C.

De préférence, la mise en contact de l'élément avec le plasma se fait pendant une très courte durée, i.e. d'environ 5 à 60 secondes.

Le procédé de séchage de canaux d'un endoscope selon l'invention comprend de préférence les étapes suivantes :
a) branchement des canaux de l'endoscope, notamment via une connectique spécifique étanche, à une unité de séchage,
b) injection d'un gaz neutre, de préférence d'air, dans les canaux d'endoscope pendant une durée comprise entre 10 et 60 secondes, le débit du gaz étant compris entre 1 et 20 1/min, le gaz étant injecté à une température comprise entre 10°C et 30°C, notamment pour éliminer l'eau résiduelle par un écoulement laminaire, puis
c) séchage des canaux de l'endoscope pendant une durée comprise entre 30 et 150 secondes, par injection du même gaz neutre qu'en b), à un débit compris entre 20 et 100 1/min, le gaz étant injecté à une température comprise entre 30°C et 60°C, notamment afin d'assurer un écoulement turbulent de l'eau résiduelle.

En outre, le procédé selon l'invention peut comprendre, après l'étape c), les étapes suivantes :
d) débrancher l'endoscope obtenu à l'étape c) de l'unité de séchage, et le placer dans un récipient étanche à l'air ;
e) injection d'un plasma généré par une décharge électrique dans un flux de diazote ou d'air dans le récipient étanche à l'air, puis fermeture dudit récipient.

Le récipient étanche à l'air est typiquement un sac plastique dont la fermeture est étanche à l'air, et comprenant une ouverture pour injection d'un gaz. Un tel sac est commercialisé par PlasmaBiotics sous la référence plasmaBAG.

L'endoscope est ainsi débranché de l'unité de séchage à l'étape d), et placé dans un tel récipient.

Ensuite, un plasma est injecté dans un tel récipient, par exemple pendant une durée de quelques secondes, de préférence une durée comprise entre 3 et 10 secondes. C'est l'étape e). Cette étape permet de désinfecter l'air contenu à l'intérieur du récipient contenant l'endoscope. Une fois fermé, l'endoscope contenu dans le récipient peut être stocké pendant une durée au moins égale à 24h, de préférence au moins égale à 48h, de préférence au moins égale à 72h.

Ce procédé, comprenant les étapes d) et e), est un procédé de séchage et de stockage d'un endoscope. Il permet le stockage de l'endoscope dans des conditions optimales, notamment pendant plusieurs jours (i.e. au moins 2 jours, de préférence au moins 3 jours), ce qui assure une innocuité optimale. Cela est notamment démontré dans les exemples.

L'invention va maintenant être exemplifiée à l'aide des exemples qui suivent, qui ne sont pas limitatifs.

### Exemple 1:

Les tests suivants sont réalisés, pour comparer la méthode de séchage selon l'invention aux méthodes classiques avec enceintes de séchage d'endoscopes thermosensibles (ESET).

Deux endoscopes sont utilisés pour réaliser ces tests :
- un Fujinon EC530 ; et
- un Olympus CF20HL.

Les temps de séchage sont indiqués dans le tableau suivant:

| **Type d'endoscope** | **Temps de séchage par la méthode selon l'invention** | **Temps de séchage en ESET (comparatif)** |
|---|---|---|
| FUJINON EC530 | 2 min et 15 sec | 90 min |
| Olympus CF20HL | 2 min et 15 sec | 60 min |

La méthode selon l'invention permet donc un séchage environ 25 à 40 fois plus rapide.

L'effet biocide du plasma d'azote, incorporé dans la méthode selon l'invention, est mis en évidence sur des tubes de 3 m de longueur :

| **Germes** | **Tube de 4 mm de diamètre** | **Tube de 2,5 mm de diamètre** | **Tube de 1,5 mm de diamètre** |
|---|---|---|---|
| P. aeruginosa, séchage plasma N2 | 5 log | 4,6 log | 4,2 log |
| P. aeruginosa, séchage N2 | 3,5 | 3,5 | 3,5 |

### Exemple 2 : évaluation de l'efficacité du procédé de séchage des canaux internes de plusieurs endoscopes selon l'invention

L'objectif de l'étude est d'évaluer la capacité de séchage des canaux internes de plusieurs endoscopes par le procédé selon l'invention.

Cette étude utilise la clause 6.2.3. de la norme NF S098-030, relative au séchage d'endoscopes dans des enceintes (ESET).

Bien que le procédé de séchage selon l'invention ne puisse être considéré comme une enceinte, les objectifs de ce procédé peuvent s'apparenter à ceux d'une ESET.

### 1) Matériels & Méthodes :

A la fin du cycle de nettoyage/désinfection, les canaux des endoscopes sont purgés et un essuyage des orifices (orifices aspiration, air/eau et biopsie) est effectué. Les canaux sont ensuite connectés à l'unité de séchage Typhoon (PlasmaBiotics) (étape a)) puis soumis au procédé de séchage selon l'invention (étapes b) et c) : insufflation d'azote puis traitement au plasma d'azote).

Conformément à la norme NF S098-030, une fois le cycle de séchage réalisé, de l'air comprimé de qualité médicale à une pression de 105 à 120 kPa est soufflé dans chaque canal de l'endoscope à tour de rôle, avec l'extrémité distale de l'endoscope positionnée entre 50mm et 100mm au-dessus et à la perpendiculaire d'un papier crépon de couleur. L'efficacité de la phase de séchage est considérée comme satisfaisante si aucune gouttelette d'humidité n'est visible sur le papier crépon.

Les conditions opératoires spécifiques du séchage sont les suivantes :

| **Nature du cycle selon le type d'endoscope** | **Durée du sèchage** | **1^{er} maximale à l'entrée** de **l'endoscope** |
|---|---|---|
| Gastroscope | 135 secondes | 45°C |
| Coloscope | | |
| Duodenoscope | | |
| Echoendoscope | | |
| Bronchoscope | 90 secondes | 45° C |
| Bronchoscope pédiatrique | 60 secondes | 40° C |

Les endoscopes testés sont les suivants :
   a) Olympus :
      Coloscopes : CF Q160 I, CF Q180 AI,
      Gastroscopes: GIF Q160, GIF Q180.
   b) Fujinon :
      Coloscopes : EC250WM, EC450WM5-H et EC250WM5,
      Gastroscopes: EG410HRS, EG250WR5,
      Duodénoscope: ED410XT.
   c) Pentax :
      Coloscopes : EC3880FK, EC380MK,
      Gastroscope: EG2940K,
      Bronchoscope: FB15V.

### 2) Résultats :

Tous les résultats des essais de séchage réalisés sur les coloscopes, gastroscopes, duodénoscope et bronchoscope ne montrent **aucune trace d'humidité.**

Au regard de ces résultats, il est conclu que le procédé de séchage selon l'invention présente une efficacité de séchage équivalente aux ESET dans la norme NF S098-030.

### Exemple 3 : évaluation du procédé de séchage selon l'invention sur la qualité microbiologique des canaux d'endoscopes

L'objectif de l'étude est d'évaluer l'effet du procédé selon l'invention sur la qualité microbiologique des canaux d'endoscopes, en comparaison avec un procédé de séchage standard (séchage manuel à air).

Cette étude utilise la clause 4.2.4. de la norme NF S098-030, relative au séchage d'endoscopes dans des enceintes (ESET).

Bien que le procédé de séchage selon l'invention ne puisse être considéré comme une enceinte, les objectifs de ce procédé peuvent s'apparenter à ceux d'une ESET.
1) Matériels & Méthodes :
Le procédé utilisé est identique à celui du point 1 de l'exemple 2.
Les conditions opératoires spécifiques du séchage sont les suivantes :

| **Nature du cycle selon le type d'endoscope** | **Durée du traitement** | 1^{er} **maximale a̋ l'entrée de l'endoscope** |
|---|---|---|
| Coloscope | 135 secondes | 45° C |

L'endoscope testé est le Pentax : Coloscope : EC3880FK.

Souches microbiennes :
*P.aeruginosa* CIP103467
Diluant des suspensions microbiennes : tryptone sel (OXOID, TV5016D).

### Solution de prélèvement :

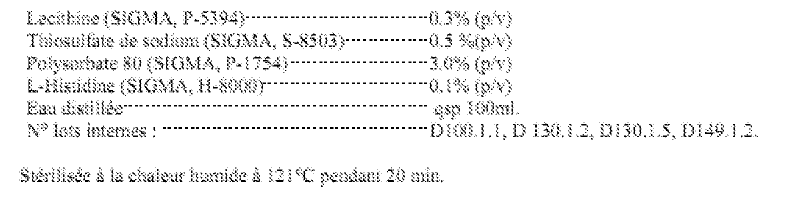

Milieu d'entretien et de dénombrement : tryptone soja (OXOID CM0131).

Le prélèvement est effectué par injection de 50 ml de solution de prélèvement via l'adaptateur de nettoyage (prélèvement des canaux air/eau), 50 ml via le raccord d'aspiration (prélèvement du canal aspiration/biopsie), 20 ml via l'entrée du canal water-jet et 50 ml via l'orifice du canal opérateur.

Les 4 volumes sont récupérés au niveau de l'extrémité distale, et ont analysés par dilution/inclusion et filtration sur membrane 0.45 µm. Les membranes sont déposées sur gélose et incubées 48h à 37°C. Après incubation, les colonies sont dénombrées et les résultats exprimés en nombre de microorganismes viable par endoscope.

L'endoscope est contaminé en injectant 15 ml de suspension microbienne d'essai (souche dans son diluant) contenant entre 1,5.10⁴ UFC/ml et 5.10⁴ UFC/ml à raison de 6 ml via le raccord d'aspiration, 6 ml via la buse d'air et 3 ml pour le canal water-jet. Après 30 min d'incubation, les canaux sont purgés avec 50 ml d'air puis maintenus à température ambiante pendant 30 min.

### 2) Résultats :

Les résultats sont présentés ci-dessous :

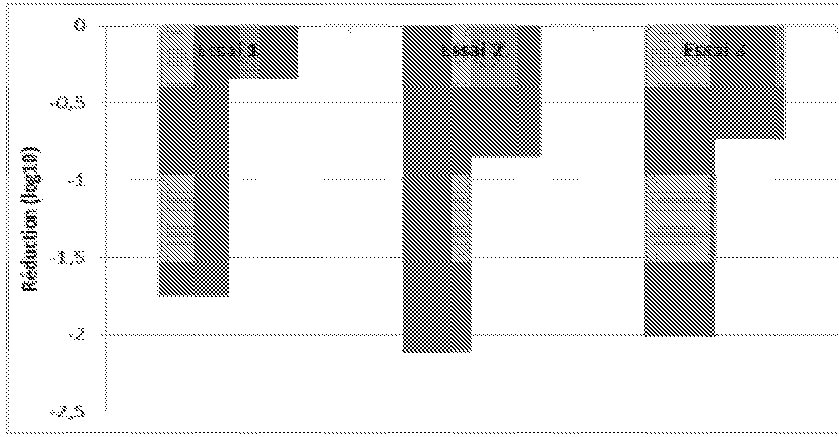

Figure : évolution de la contamination microbienne interne du coloscope soumis au séchage selon l'invention (2^{ème} série de colonnes), par rapport au niveau de contamination soumis au procédé standard (soufflage à l'air médical, 1^{ère} série de colonnes).

Les résultats montrent que le taux de contamination des canaux internes de l'endoscope séché selon l'invention :
- reste inférieur au niveau de contamination initial de l'endoscope avant séchage ; et
- est toujours inférieur à celui de l'endoscope soumis au procédé standard.

### Exemple 4 : évaluation de l'efficacité du procédé de séchage selon l'invention, incluant le stockage

L'objectif est d'évaluer les capacités du procédé de séchage et stockage selon l'invention (étapes a) à e)) à maintenir la qualité microbiologique des endoscopes, selon une méthodologie inspirée de la norme NF EN 16442 : 2015 (norme ESET).

### Matériel :

- Endoscopes FUJINON EC 250 WM
- Plasma Typhoon (Plasmabiotics)
- Kit de connexion pour connecter les endoscopes au Plasma Typhoon
- Plasma Bag (sacs de stockage d'endoscopes)

### 1. Etape 1 : Préparation des endoscopes

La procédure de préparation de l'endoscope est identique pour tous les endoscopes analysés:
1. Soumettre l'endoscope à un cycle standard de nettoyage/désinfection
2. Contaminer l'endoscope artificiellement en injectant dans chacun des canaux une solution de contamination contenant environ 1.5.10³ à 5.10³ *Pseudomonas aeruginosa*/*ml*
3. Maintenir l'endoscope à température ambiante pendant 30 min
4. Purger les canaux d'endoscope afin d'éliminer tout excès de solution de contamination
5. Maintenir l'endoscope à température ambiante (pendant 1h ou selon les instructions du fabricant).

Après la période d'incubation, l'endoscope est soumis au procédé de séchage et de stockage selon l'invention. Au total :
- 3 essais sont réalisés en prélevant l'endoscope juste après la purge afin de déterminer le niveau de contamination des endoscopes avant séchage et stockage (contrôle) ;
- 2 essais sont réalisés en prélevant l'endoscope après 24h, 48h et 72h de stockage selon le procédé à tester (essai selon l'invention) ; et
- 2 essais sont réalisés en prélevant l'endoscope après 24h, 48h et 72h de stockage à l'extérieur (procédé de stockage standard).

### Procédé de séchage et de stockage selon l'invention

### 2. Etape 2 : séchage des endoscopes (étapes a) à c))

Le procédé de séchage est réalisé en utilisant Plasma Typhoon :
En début des tests :
   1. Ouvrir la bouteille de gaz, régler la pression à 3 bars. En cas d'utilisation d'air médical, régler la pression à 3 bars.
   2. Mettre Plasma Typhoon sous-tension (ON)

Pour chaque cycle de séchage :
3. Connecter tous les canaux d'endoscope aux sorties plasma du Plasma Typhoon en utilisant le kit de connexion:
   a. Suction/Operating channel
   b. Air/Water channel
   c. Water jet channel
4. Mettre le pont au niveau de l'extrémité proximale de l'endoscope (dans le cas de gastroscope, coloscope, duodénoscope, échoendoscope)
5. Démarrer le cycle de séchage
6. Une fois le cycle complété, déconnecter l'endoscope.

### 3. Etape 3 : stockage des endoscopes (étapes d) et e))

Une fois séchés, les endoscopes sont stockés dans des sacs en polyéthylène :
1. Placer l'endoscope dans le sac en polyéthylène (Plasma Bag)
2. Fermer le sac en utilisant le ZIP sur le côté
3. Connecter Plasma Typhoon au luer placé dans le coin du sac
4. Choisir le cycle « Stockage »
5. Démarrer le cycle « Stockage » qui sert à souffler du plasma dans le sac pendant 5 secondes
6. Une fois le cycle complété, déconnecter le Plasma Typhoon du sac (luer) et fermer le sac avec un bouchon luer

### Analyse :

L'efficacité du procédé de stockage est déterminée en comparant pour chacun des temps de contact le niveau de contamination des endoscopes stockés selon le procédé avec un endoscope non-soufflé au Typhoon et gardé à l'extérieur.

Les résultats sont les suivants :

| **Essai** | **Temps de stockage** | **Dénombrement (UFC/ml)** |
|---|---|---|
| Selon l'invention | 24h | 1 |
| Standard | 24h | 24.10⁷ |

| | | |
|---|---|---|
| Selon l'invention | 48h | 0 |
| Standard | 48h | 1,9.10⁹ |
| Selon l'invention | 72h | 0 |
| Standard | 72h | 3,1.10⁹ |

Le procédé de séchage et de stockage selon l'invention permet ainsi un maintien de la qualité microbiologique de l'endoscope pendant une durée d'au moins 72h.

## Revendications

1. Procédé de séchage de canaux d'un endoscope, comprenant les étapes suivantes :
a) branchement de l'endoscope, notamment via une connectique spécifique de préférence étanche, à une unité de séchage,
b) injection d'un gaz neutre dans les canaux d'endoscope pendant une durée comprise entre 10 et 60 secondes, le débit du gaz étant compris entre 1 et 20 1/min, le gaz étant injecté à une température comprise entre 10°C et 30°C, pour éliminer le liquide résiduel par un écoulement laminaire du gaz, puis
c) séchage des canaux de l'endoscope pendant une durée comprise entre 30 et 150 secondes, par injection d'un gaz à une température comprise entre 30°C et 60°C, le débit du gaz étant compris entre 20 1/min et 100 1/min, pour générer un écoulement turbulent du gaz.

2. Procédé selon la revendication 1, **caractérisé en ce que** le gaz utilisé dans l'étape c) est neutre, ou est un plasma généré par une décharge électrique dans un flux de diazote ou d'air.

3. Procédé selon la revendication 1 ou 2, **caractérisé en ce que** le gaz neutre est du diazote ou de l'air.

4. Procédé selon la revendication 2, **caractérisé en ce que** le plasma est obtenu par l'activation par un champ électrique, à pression atmosphérique, du flux de diazote ou d'air.

5. Procédé selon l'une des revendications 2 ou 4, **caractérisé en ce que** le plasma est un plasma froid obtenu en zone de post-décharge.

6. Procédé selon l'une des revendications 1 à 5, **caractérisé en ce que** le séchage c) se fait par injection du gaz dans les canaux de l'endoscope par la cage à pistons, ou par son extrémité proximale.

7. Procédé selon l'une des revendications 2, 4 ou 5, **caractérisé en ce que** le plasma est obtenu, dans l'unité de séchage, par les étapes suivantes :
- passage d'un flux de diazote ou de l'air, ayant un débit compris entre 1 l/min et 100 1/min, dans l'unité ; puis
- soumission du flux obtenu à une décharge électrique.

8. Procédé selon l'une des revendications 2, 4, 5 ou 7, **caractérisé en ce que** le plasma est utilisé à une température comprise entre 30 et 50°C.

9. Procédé selon l'une des revendications 1 à 8, **caractérisé en ce que** la durée globale des étapes a) à c) est comprise entre 1 et 5 minutes.

10. Procédé selon l'une des revendications 1 à 9, **caractérisé en ce que** l'endoscope est désinfecté chimiquement préalablement aux étapes a) à c).

11. Procédé selon l'une des revendications 1 à 10, **caractérisé en ce qu'**il comprend, après l'étape c), les étapes suivantes :
d) débrancher l'endoscope obtenu à l'étape c) de l'unité de séchage, et le placer dans un récipient étanche à l'air ;
e) injection d'un plasma généré par une décharge électrique dans un flux de diazote ou d'air dans le récipient étanche à l'air, puis fermeture dudit récipient.

## Patentansprüche

1. Verfahren zum Trocknen der Kanäle eines Endoskops, das die folgenden Schritte umfasst:
a) Anschließen des Endoskops, insbesondere über einen speziellen, vorzugsweise dichten Anschluss, an eine Trocknungseinheit,
b) Einblasen eines Neutralgases in die Endoskopkanäle über eine Dauer zwischen 10 und 60 Sekunden, wobei die Durchflussmenge des Gases zwischen 1 und 20 l/min beträgt, wobei das Gas mit einer Temperatur zwischen 10 °C und 30 °C eingeblasen wird, um die Restflüssigkeit durch eine laminare Strömung des Gases auszutragen, und anschließend
c) Trocknen der Kanäle des Endoskops über eine Dauer zwischen 30 und 150 Sekunden durch Einblasen eines Gases mit einer Temperatur zwischen 30 °C und 60 °C, wobei die Durchflussmenge des Gases zwischen 20 1/min und 100 1/min beträgt, um eine turbulente Strömung des Gases zu erzeugen.

2. Verfahren nach Anspruch 1, **dadurch gekennzeichnet, dass** das in Schritt c) verwendete Gas neutral ist, oder ein Plasma ist, das durch eine elektrische Entladung in einem Distickstoff- oder Luftstrom erzeugt wird.

3. Verfahren nach Anspruch 1 oder 2, **dadurch gekennzeichnet, dass** es sich bei dem Neutralgas um Distickstoff oder Luft handelt.

4. Verfahren nach Anspruch 2, **dadurch gekennzeichnet, dass** das Plasma durch die Aktivierung des Distickstoff- oder Luftstroms bei Atmosphärendruck durch ein elektrisches Feld erhalten wird.

5. Verfahren nach einem der Ansprüche 2 oder 4, **dadurch gekennzeichnet, dass** es sich bei dem Plasma um ein kaltes Plasma handelt, das im Nach-Entladungsbereich erhalten wird.

6. Verfahren nach einem der Ansprüche 1 bis 5, **dadurch gekennzeichnet, dass** das Trocknen c) durch Einblasen des Gases in die Kanäle des Endoskops durch das Ventilgehäuse oder durch sein proximales Ende erfolgt.

7. Verfahren nach einem der Ansprüche 2, 4 oder 5, **dadurch gekennzeichnet, dass** das Plasma in der Trocknungseinheit durch die folgenden Schritte erhalten wird:
- Einleiten eines Distickstoff- oder Luftstroms mit einer Durchflussmenge zwischen 1 l/min und 100 l/min in die Einheit; und anschließend
- Anlegen einer elektrischen Entladung an den erhaltenen Strom.

8. Verfahren nach einem der Ansprüche 2, 4, 5 oder 7, **dadurch gekennzeichnet, dass** das Plasma bei einer Temperatur zwischen 30 und 50 °C verwendet wird.

9. Verfahren nach einem der Ansprüche 1 bis 8, **dadurch gekennzeichnet, dass** die Gesamtdauer der Schritte a) bis c) zwischen 1 und 5 Minuten beträgt.

10. Verfahren nach einem der Ansprüche 1 bis 9, **dadurch gekennzeichnet, dass** das Endoskop vor den Schritten a) bis c) chemisch desinfiziert wird.

11. Verfahren nach einem der Ansprüche 1 bis 10, **dadurch gekennzeichnet, dass** es nach Schritt c) die folgenden Schritte umfasst:
d) Abtrennen des in Schritt c) erhaltenen Endoskops von der Trocknungseinheit und Einlegen desselben in einen luftdichten Behälter;
e) Einblasen eines durch eine elektrische Entladung in einem Distickstoff- oder Luftstrom erzeugten Plasmas in den luftdichten Behälter, und anschließend Verschließen des Behälters.

## Claims

1. Method for drying channels of an endoscope, the method comprising the following steps:
a) connecting the endoscope, in particular via a specific, preferably watertigh, connector, to a drying unit,
b) injecting a neutral gas into the endoscope channels for a period of time between 10 and 60 seconds, the flow rate of the gas being between 1 and 20 l/min, the gas being injected at a temperature between 10°C and 30°C, in order to remove the residual liquid by means of a laminar flow of the gas, then
c) drying the channels of the endoscope for a period of time between 30 and 150 seconds by injecting a gas at a temperature between 30°C and 60°C, the flow rate of the gas being between 20 l/min and 100 1/min, in order to generate a turbulent flow of the gas.

2. The method according to claim 1, **characterized in that** the gas used in step c) is neutral, or it is a plasma generated by an electrical discharge in a stream of dinitrogen or air.

3. The method of claim 1 or 2, wherein the neutral gas is dinitrogen or air.

4. The method of claim 2, wherein the plasma is obtained by electric field activation, at atmospheric pressure, of the stream of dinitrogen or air.

5. The method according to any one of claims 2 or 4, wherein the plasma is a cold plasma obtained in a post-discharge zone.

6. The method according to any one of claims 1 to 5, **characterized in that** the drying c) is performed by injecting the gas into the channels of the endoscope through the piston cage, or through its proximal end.

7. The method according to any one of claims 2, 4 or 5, **characterized in that** the plasma is obtained, in the drying unit, via the following steps:
- passage of a flow of dinitrogen or air, having a flow rate comprised between 1 l/min and 100 1/min, through the unit; then
- submission of the resulting flow to an electric discharge.

8. The method according to any one of claims 2, 4, 5 or 7, **characterized in that** the plasma is used at a temperature between 30 and 50°C.

9. The method according to any one of claims 1 to 8, **characterized in that** the overall duration of steps a) to c) is between 1 and 5 minutes.

10. The method according to any of claims 1 to 9, **characterized in that** the endoscope is chemically disinfected prior to steps a) to c).

11. The method according to any of claims 1 to 10, **characterized in that** it comprises, after step c), the following steps:
d) disconnecting the endoscope obtained in step c) from the drying unit, and placing it in an airtight container;
e) injecting a plasma generated by an electric discharge into a flow of dinitrogen or air in the airtight container, and then closing said container.
